(19) Europäisches Patentamt
European Patent Office
Office européen des brevets

(11) Veröffentlichungsnummer : **0 326 854 B1**

(12) # EUROPÄISCHE PATENTSCHRIFT

(45) Veröffentlichungstag der Patentschrift : **03.06.92 Patentblatt 92/23**

(51) Int. Cl.$^5$ : **C07C 43/11,** C07C 43/10, C07C 41/18, C08G 65/32

(21) Anmeldenummer : **89100766.8**

(22) Anmeldetag : **18.01.89**

(54) **Verfahren zur Herstellung von Alkylenglykoldialkylethern.**

(30) Priorität : **30.01.88 DE 3802783**

(43) Veröffentlichungstag der Anmeldung : **09.08.89 Patentblatt 89/32**

(45) Bekanntmachung des Hinweises auf die Patenterteilung : **03.06.92 Patentblatt 92/23**

(84) Benannte Vertragsstaaten : **AT BE CH DE FR GB IT LI NL SE**

(56) Entgegenhaltungen :
**EP-A- 0 075 936**
**EP-A- 0 076 461**

(56) Entgegenhaltungen :
**CHEMICAL ABSTRACTS, Band 83, Nr. 1, 7. Juli 1975, Seite 762, Zusammenfassung Nr. 9186m, Columbus, Ohio, US; & JP-A-74 25246 (MITSUI TOATSU CHEMICALS, INC.) 28-06-1974**

(73) Patentinhaber : **HOECHST AKTIENGESELLSCHAFT**
**Postfach 80 03 20**
**W-6230 Frankfurt am Main 80 (DE)**

(72) Erfinder : **Stankowiak, Achim, Dr.**
**Ortlehnerstrasse 20**
**W-8269 Burgkirchen (DE)**
Erfinder : **Schulz, Hildegard**
**Graf-Lehnberger-Strasse 2**
**W-8343 Triftern (DE)**

**EP 0 326 854 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Alkylenglykoldialkylethern aus Alkylenglykolmonoalkylethern mit einer sekundären Hydroxylgruppe.

Die Herstellung von Alkylenglykoldialkylethern aus den entsprechenden Monoalkylethern erfolgt im allgemeinen nach dem schon seit langem bekannten Williamson-Verfahren oder einer davon. Bei all diesen Verfahren wird von einem Variante Alkylenglykolmonoalkylether ausgegangen, dessen eine im allgemeinen endständige Hydroxylgruppe, die primärer, sekundärer oder tertiärer Art sein kann, mit einem Alkylierungsmittel verethert wird. Im einzelnen wird bei diesem Verfahren der eingesetzte Alkylenglykolmonoalkylether zunächst mit Alkali zur Glykolatverbindung umgesetzt. Das Glykolat wird dann mit Alkylhalogenid, Dialkylsulfat oder einem anderen geeigneten Alkylierungsmittel in den angestrebten Diether übergeführt. Diese schon seit langem bekannten Verfahren zur technischen Herstellung von Alkylenglykoldialkylethern aus Alkylenglykolmonoalkylethern mit einer primären, sekundären oder tertiären Hydroxylgruppe: weisen neben der Mehrstufigkeit auch noch weitere Nachteile auf. So fallen hohe Mengen an nur schwer verwertbarem Alkalimetallhalogenid an.

Es ist auch schon bekannt, Verbindungen mit einer oder mehreren primären, sekundären oder tertiären Hydroxylgruppen durch katalytische Hydrierung in die entsprechenden hydroxylgruppenfreien Verbindungen überzuführen oder in solche Verbindungen, die weniger Hydroxylgruppen enthalten als die Ausgangsverbindungen. Diese Hydrierungen werden in der Regel mit Wasserstoff unter einem mehr oder weniger großen Druck und mit Raney-Nickel, Kobalt, Kupfer, Chrom, Molybdän, Palladium, Platin, Ruthenium und dergleichen als Katalysator durchgeführt. So wird in der US-Patentschrift 4 649 225 die Hydrierung von Alkylenglykolen, wie Diethylenglykol oder Triethylenglykol, zu den entsprechenden Ethylenglykolmonomethyl- und/oder Ethylenglykolmonoethylethern unter Verwendung von Iridium als Katalysator beschrieben. Die eingesetzten, primäre Hydroxylgruppen enthaltenden Alkylenglykole werden an der endständigen Kohlenstoff-Kohlenstoff-Bindung oder an der endständigen Kohlenstoff-Sauerstoff-Bindung gespalten, wobei im ersten Fall der Monomethylether und im zweiten Fall der Monoethylether, neben Monoethylenglykol und Ethanol als Nebenprodukte, gebildet werden.

Es wurde nun überraschenderweise gefunden, daß bestimmte sekundäre Hydroxylgruppen bei Verwendung von Nickel-Trägerkatalysatoren mit Wasserstoff bei Normaldruck hydriert werden können und dabei im wesentlichen nur die gewünschten Verbindungen gebildet werden. Gefunden wurde demnach ein Verfahren zur Herstellung von Alkylenglykoldialkylethern aus Alkylenglykolmonoalkylethern mit einer sekundären Hydroxylgruppe, das dadurch gekennzeichnet ist, daß ein Alkylenglykolmonoalkylether der nachstehenden Formel I

$$R^1O(CH_2\underset{R^2}{\underset{|}{CHO}})_n-CH_2-\underset{R^3}{\underset{|}{CHOH}}$$

worin $R^1$ ein Alkylrest mit 1 bis 25 C-Atomen ist, $R^2$ H, $CH_3$ oder $C_2H_5$ ist und innerhalb der Kette des Polyoxalkylenrestes, statistisch oder in Blöcken angeordnet, auch alle drei Bedeutungen annehmen kann, $R^3$ $CH_3$ oder $C_2H_5$ ist und n 1 bis 100 bedeutet,
in Gegenwart von Nickel auf einem Träger als Katalysator mit Wasserstoff bei Atmosphärendruck umgesetzt und aus dem Umsetzungsprodukt der gebildete Alkylenglykoldialkylether gewonnen wird.

Beim erfindungsgemäßen Verfahren werden also spezielle Alkylenglykolmonoalkylether zu den entsprechenden Diethern hydriert, und zwar solche Monoether, deren eine endständige Hydroxylgruppe eine sekundäre Hydroxylgruppe an einem Isopropylrest oder an einem sekundären Butylrest ist. Diese Hydroxyalkylreste, die sich am Ende der Alkylenglykolkette befinden, werden zum n-Propylrest ($C_3H_7$) und zum n-Butylrest ($C_4H_9$) hydriert. Die mit dem erfindungsgemäßen Verfahren erhaltenen Diether entsprechen somit den nachstehenden Formeln

$$R^1O(CH_2\underset{\displaystyle R^2}{\underset{|}{C}}HO)_n-CH_2CH_2CH_3 \qquad \text{und}$$

$$R_1O(CH_2\underset{\displaystyle R^2}{\underset{|}{C}}HO)_n-CH_2CH_2CH_2CH_3,$$

worin $R^1$, $R^2$ und n die angegebenen Bedeutungen haben.

Nachdem die bekannten Hydrierungen von primären, sekundären oder tertiären Hydroxylgruppen in der Regel unter Druck und im Falle der Verwendung von Nickel als Katalysator mit Raney-Nickel durchgeführt werden, ist es ein unerwartetes Ergebnis, daß die Hydrierung der in Rede stehenden Alkylenglykolmonoalkylether nur bei Atmosphärendruck und mit einem Nickel-Trägerkatalysator zum Erfolg führt.

Die beim erfindungsgemäßen Verfahren einzusetzenden Alkylenglykolmonoalkylether sind bekannt und im Handel erhältlich. Bevorzugte Monoether sind solche, wenn $R^1$ in Formel I ein Alkylrest mit 4 bis 18 C-Atomen ist, $R^2$ H oder $CH_3$ ist und - wie oben bereits erwähnt - innerhalb der Kette des Polyoxalkylenrestes, statistisch oder in Blöcken angeordnet,auch beide Bedeutungen annehmen kann, $R^3 = CH_3$ und n = 5 bis 50 ist. Beim Index n kann es sich um ganze oder gebrochene Zahlen handeln. Die einzusetzenden Alkylenglykolmonoether werden bekanntlich durch Oxalkylierung der dem Rest $R^1O$ entsprechenden Alkohole erhalten. Wird der Alkohol mit Ethylenoxid allein oder mit Ethylenoxid als letztem Oxalkylierungsagens umgesetzt, so ist, wie aus Formel I hervorgeht, auch noch mindestens eine Propylenoxid-Einheit oder Butylenoxid-Einheit anzufügen, damit ein Alkylenglykolmonoalkylether mit der in Rede stehenden sekundären Hydroxylgruppe vorliegt; das ist nämlich das entscheidende Merkmal des Ausgangsmonoethers gemäß Erfindung. Bei den zur Oxalkylierung eingesetzten Alkoholen kann es sich gemäß den Bedeutungen von $R^1$ um einzelne Alkohole oder um Alkoholgemische mit vorzugsweise 4 bis 18 C-Atomen handeln. Bei Einsatz von Alkoholen mit einer relativ hohen Anzahl von C-Atomen sind die verfügbaren technischen Produkte, wie Cocosfettalkohol, Talgfettalkohol und dergleichen bevorzugt (in diesen Fettalkoholen liegen bekanntlich Alkylreste mit im wesentlichen 8 bis 18 C-Atomen vor; die Anwesenheit von Alkenylresten in diesen Fettalkoholen hat keinen Einfluß auf die erfindungsgemäße Hydrierung).

Die Hydrierung der erfindungsgemäß einzusetzenden Alkylenglykolmonoalkylether erfolgt mit Hilfe eines Nickel-Trägerkatalysators. Solche Nickel-Katalysatoren sind bekannt und im Handel erhältlich. Sie bestehen im allgemeinen aus 5 bis 80 Gew.-% Nickel, zweckmäßigerweise 30 bis 65 Gew.-% Nickel, auf einem Trägermaterial, Gewichtsprozente bezogen auf den gesamten Katalysator. Das Trägermaterial ist nicht kritisch. Geeignete inerte Trägermaterialien sind Aluminiumoxide, Kohle, Kieselgur, Siliciumdioxid, Siliciumcarbid, Zeolithe, Metalloxide und ähnliche. Sie werden charakterisiert durch die spezifische Oberfläche, das Porenvolumen und den mittleren Porendurchmesser. Die Trägermaterialien werden in der Regel in Form von Pulver, Granulat, Kugeln oder Ringen eingesetzt. Die einzusetzende Katalysatormenge kann in weiten Grenzen variieren. Mit weniger als 0,5 Gew.-% Nickel, bezogen auf Alkylenglykolmonoalkylether, verläuft die Hydrierung nur sehr langsam und größere Nickelmengen als 15 Gew.-% sind im allgemeinen nicht mehr wirtschaftlich. In der Regel wird also soviel Nickel-Trägerkatalysator eingesetzt, daß 0,5 bis 15 Gew.-% Nickel, vorzugsweise 1 bis 10 Gew.-% Nickel, bezogen auf die zu hydrierende Menge an Alkylenglykolmonoalkylether, vorliegen.

Die erfindungsgemäße Umsetzung der angegebenen Alkylenglykolmonoalkylether mit Wasserstoff wird bei Atmosphärendruck, das heißt ohne Wasserstoffüberdruck, durchgeführt. Dies kann kontinuierlich oder diskontinuierlich erfolgen. Nach einer bevorzugten Vorgangsweise wird der zu hydrierende Glykolmonoether und der Nickel-Trägerkatalysator, vorzugsweise in Pulverform, in einem Reaktionsgefäß mit Rückflußkühler vorgelegt. Die Mischung wird auf Reaktionstemperatur erhitzt und bei dieser Temperatur zweckmäßigerweise unter Rühren mit überschüssigem Wasserstoff (die stöchiometrische Menge an Wasserstoff beträgt 1 mol pro mol Glykolmonoether)drucklos in Kontakt gebracht. Dies wird vorzugsweise so durchgeführt, daß der Wasserstoff durch die auf Reaktionstemperatur gehaltene Mischung hindurchgeleitet und der überschüssige Wasserstoff durch den Rückflußkühler, in dessen Wasserabscheider sich das Reaktionswasser sammelt, abgezogen wird. Wenn auch das Mengenverhältnis von Glykolmonoether zu Wasserstoff in weiten Grenzen variieren kann, werden im allgemeinen 30 bis 500 Liter Wasserstoff pro Kilogramm Glykolmonoether pro Stunde, vorzugsweise 50 bis 250 Liter Wasserstoff pro Kilogramm Glykolmonoether pro Stunde, eingesetzt. Mit weniger als den genannten 30 Liter Wasserstoff werden auch bei höherer Reaktionstemperatur und relativ viel Katalysator sehr lange Reaktionszeiten benötigt und mehr Wasserstoff als die genannten 500 Liter ist nicht mehr wirtschaftlich. Die Reaktionstemperatur liegt bei 150 bis 300 °C, vorzugsweise 180 bis 250 °C. Die Reaktionszeit bis zur Erreichung einer praktisch vollständigen Hydrierung des Alkylenglykolmonoalkylethers zum Diether liegt im Bereich von 5 bis 20 Stunden. Die Hydrierung wird zweckmäßigerweise durch laufende Bestimmung der Hydroxylzahl des Reaktionsproduktes verfolgt. Nach Erreichung der angestrebten niedrigen Hydroxylzahl im Vergleich zur hohen Hydroxylzahl des eingesetzten Glykolmonoethers wird die Hydrierung beendet. Im flüssigen, mehr oder weniger viskosen Reaktionsprodukt liegt der angestrebte Glykoldiether vor. Zur Gewinnung von reinem Glykoldiether wird das Reaktionsprodukt von Katalysator, gegebenenfalls nicht umgesetztem Glykolmonoether und gegebenenfalls gebildeten Nebenprodukten befreit. So kann der Nickel-Trägerkatalysator einfach durch Filtration von dem flüssigen Reaktionsprodukt abgetrennt werden. Die mit dem erfindungsgemäßen Verfahren erhaltenen Alkylenglykoldialkylether stellen bekanntlich farblose, mehr oder weniger viskose Flüssigkeiten dar. Sie werden in hoher Ausbeute erhalten. Nebenprodukte liegen, wenn überhaupt, nur sehr wenig vor.

Die Erfindung wird nun an Beispielen noch näher erläutert. In Vergleichsbeispielen wird gezeigt, daß die Hydrierung der angegebenen Glykolmonoether zu den entsprechenden Diethern erfolglos verläuft, wenn sie nicht mit einem Nickel-Trägerkatalysator, sondern beispielsweise mit Raney-Nickel, einem Kobalt-Trägerkatalysator, Palladium-Trägerkatalysator oder einem Platin-Trägerkatalysator duchgeführt wird, und wenn sie nicht bei Atmosphärendruck, sondern unter einem mehr oder weniger großen Wasserstoffdruck durchgeführt wird.

Beispiel 1

Dieses und alle weiteren Beispiele wurden in einem Reaktionsgefäß durchgeführt, das mit einem Rührer, Thermometer, Gaseinleitungsrohr und Rückflußkühler mit Wasserabscheider ausgestattet war.
Es werden 500 g Propylenglykolmonobutylether der Formel

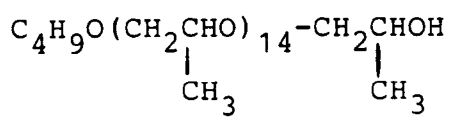

und 39 g eines pulverförmigen Nickel-Trägerkatalysators, bestehend aus 64 Gew.-% Nickel auf Aluminiumoxid/Siliciumdioxid als Trägermaterial, das sind 25 g oder 5 Gew.-% Nickel, bezogen auf die 500 g Monobutylether, vorgelegt (die für n der allgemeinen Formel I stehende Zahl 14 in obiger Formel stellt bekanntlich einen Mittelwert dar). Nach Spülung des Reaktionsgefäßes mit Stickstoff wurde die vorgelegte Mischung unter Rühren auf die Reaktionstemperatur von 220 °C erhitzt. Der Hydrierwasserstoff wurde über das obengenannte Einleitungsrohr, das in die Reaktionsmischung eintauchte, zugeführt und der Überschuß über den Rückflußkühler mit Wasserabscheider abgeführt. Es wurden 130 Liter Wasserstoff pro Kilogramm Glykolmonoether pro Stunde durch die Mischung geführt. Sobald das Reaktionsprodukt eine Hydoxylzahl von 2 aufwies (der eingesetzte Glykolmonoether hatte die Hydroxyzahl 80) wurde die Wasserstoffzufuhr beendet und das Reaktionsprodukt abgekühlt, wobei während des Abkühlens anstelle von Wasserstoff Stickstoff zum Spülen zugeführt wurde. Das Reaktionsprodukt, das vom Katalysator durch Filtrieren getrennt wurde und im wesentlichen aus dem angestrebten Propylenglykolbutylpropylether bestand, war eine klare, farblose, viskose Flüssigkeit. Aus den angegebenen Hydroxylzahlen berechnet sich eine Ausbeute an Glykoldiether von 97 Gew.-%.

Beispiel 2

Ansatz: 500 g Propylenglykolmonobutylether der Formel

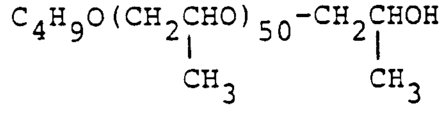

und 23 g des Nickel-Trägerkatalysators vom Beispiel 1, das sind 15 g oder 3 Gew.-% Nickel, bezogen auf die 500 g Monobutylether.
Durchführung: Reaktionstemperatur 240 °C. Wasserstoffmenge 130 Liter pro Kilogramm Glykolmonoether pro Stunde bis das Reaktionsprodukt eine Hydroxylzahl von 2 hatte (der eingesetzte Monoether hatte die Hydroxylzahl 32). Der angestrebte Propylenglykolbutylpropylether wurde in einer Ausbeute von 94 Gew.-% erhalten.

Beispiel 3

Ansatz: 500 g Propylenglykolmonoisotridecylether der Formel

$$\text{iso-}C_{13}H_{27}O(CH_2\underset{\displaystyle CH_3}{\underset{|}{C}}HO)_6-CH_2\underset{\displaystyle CH_3}{\underset{|}{C}}HOH$$

und 77 g eines pulverförmigen Nickel-Trägerkatalysators, bestehend aus 65 Gew.-% Nickel auf Kieselgur als Trägermaterial, das sind 50 g oder 10 Gew.-% Nickel, bezogen auf die 500 g Monoisotridecylether.
Durchführung: Reaktionstemperatur 190 °C. Wasserstoffmenge 50 Liter pro Kilogramm Glykolmonoether pro Stunde bis das Reaktionsprodukt eine Hydroxylzahl von 1 hatte (der eingesetzte Monoether hatte die Hydroxylzahl 89). Der Propylenglykolisotridecylpropylether wurde in einer Ausbeute von 99 Gew.-% erhalten.

Beispiel 4

Ansatz: 500 g Propylenglykolmonoisotridecylether der Formel

$$\text{iso-}C_{13}H_{27}O(CH_2\underset{\displaystyle CH_3}{\underset{|}{C}}HO)_{17}-CH_2\underset{\displaystyle CH_3}{\underset{|}{C}}HOH$$

und 62 g des Nickel-Trägerkatalysators vom Beispiel 3, das sind 40 g oder 8 Gew.-% Nickel, bezogen auf die 500 g Monoisotridecylether.
Durchführung: Reaktionstemperatur 230 °C. Wasserstoffmenge 130 Liter pro Kilogramm Glykolmonoether pro Stunde bis das Reaktionsprodukt eine Hydroxylzahl von 3 hatte (der eingesetzte Monoether hatte die Hydroxylzahl 43). Der Propylenglykolisotridecylpropylether wurde in einer Ausbeute von 93 Gew.-% erhalten.

Beispiel 5

Ansatz: 500 g Ethylenglykolmonoisotridecylether der Formel

$$\text{iso-}C_{13}H_{27}O(CH_2CH_2O)_{17}-CH_2\underset{\displaystyle CH_3}{\underset{|}{C}}HOH$$

und Nickel-Trägerkatalysator wie im Beispiel 4.
Durchführung: wie im Beispiel 4. Der Ethylenglykolisotridecylpropylether wurde in einer Ausbeute von 95 Gew.-% erhalten.

Beispiel 6

Ansatz: 500 g Ethylen/Propylenglykolmonomethylether der Formel

$$CH_3O(CH_2CH_2O)_{11}-(CH_2\underset{\displaystyle CH_3}{\underset{|}{C}}HO)_2-CH_2\underset{\displaystyle CH_3}{\underset{|}{C}}HOH$$

und 56 g eines pulverförmigen Nickel-Trägerkatalysators, bestehend aus 45 Gew.-% Nickel auf Kieselgur als Trägermaterial, das sind 25 g oder 5 Gew.-% Nickel, bezogen auf die 500 g Monomethylether.
Durchführung: Reaktionstemperatur 230°C. Wasserstoffmenge 130 Liter pro Kilogramm Glykolmonoether pro Stunde bis das Reaktionsprodukt eine Hydroxylzahl von 11 hatte (der eingesetzte Monoether hatte die Hydroxylzahl 100). Der Glykolmethylpropylether wurde in einer Ausbeute von 90 Gew.-% erhalten.

Beispiel 7

Ansatz: 500 g Tetraethylen/Isobutylenglykolmonomethylether der Formel

$$CH_3O(CH_2CH_2O)_4-CH_2\underset{\displaystyle C_2H_5}{\underset{|}{C}}HOH$$

und 110 g des Nickel-Trägerkatalysators vom Beispiel 6, das sind 50 g oder 10 Gew.-% Nickel, bezogen auf die 500 g Monomethylether.
Durchführung: Reaktionstemperatur 175 °C. Wasserstoffmenge 250 Liter pro Kilogramm Glykolmonoether pro Stunde bis das Reaktionsprodukt eine Hydroxylzahl von 25 hatte (der eingesetzte Monoether hatte die Hydroxylzahl 204). Der Tetraethylenglykolmethylbutylether wurde in einer Ausbeute von 88 Gew.-% erhalten.

Beispiel 8

Ansatz: 500 g Ethylenglykolmonococosalkylether der Formel

$$\text{Cocosalkyl}-O(CH_2CH_2O)_{10}-CH_2\underset{\displaystyle CH_3}{\underset{|}{C}}HOH$$

und Nickel-Trägerkatalysator wie im Beispiel 1.
Durchführung: Reaktionstemperatur 220°C. Wasserstoffmenge 180 Liter pro Kilogramm Glykolmonoether pro Stunde bis das Reaktionsprodukt eine Hydroxylzahl von 15 hatte (der eingesetzte Monoether hatte eine Hydroxylzahl von 82). Der Ethylenglykolcocosalkylpropylether wurde in einer Ausbeute von 82 Gew.-% erhalten.

Beispiel 9

Ansatz: 500 g Ethylen/Isobutylenglykolmonococosalkylether der Formel

$$\text{Cocosalkyl}-O(CH_2CH_2O)_{10}-CH_2\underset{\displaystyle C_2H_5}{\underset{|}{C}}HOH$$

und Nickel-Trägerkatalysator wie im Beispiel 2.
Durchführung: Reaktionstemperatur 220 °C. Wasserstoffmenge 200 Liter pro Kilogramm Glykolmonoether pro Stunde bis das Reaktionsprodukt eine Hydroxylzahl von 15 hatte (der eingesetzte Monoether hatte eine Hydroxylzahl von 80). Der Ethylenglykolcocosalkylbutylether wurde in einer Ausbeute von 81 Gew.-% erhalten.

Vergleichsbeispiele 1 bis 4

Beispiel 1 wurde viermal wiederholt, wobei als Katalysator nicht der Nickel-Trägerkatalysator, sondern Raney-Nickel und ein entsprechender Kobalt-, Palladium- und Platin-Trägerkatalysator eingesetzt wurde. In allen vier Fällen wurde keine nennenswerte Hydrierung des Monoethers zum Diether erreicht.

Vergleichsbeispiele 5 und 6

In einem Schüttelautoklaven wurden 500 g Propylenglykolmonobutylether vom Beispiel 1 und 25 g Raney-Nickel, das sind 5 Gew.-% Nickel, bezogen auf die 500 g Monobutylether, vorgelegt. Nach Spülung mit Stickstoff wurde die vorgelegte Mischung unter Schütteln auf 220 °C erhitzt und mit soviel Wasserstoff beaufschlagt, daß ein Anfangsdruck von 9 MPa (Vergleichsbeispiel 5) und 18 MPa (Vergleichsbeispiel 6) vorlagen. Auch nach 10 Stunden Reaktionszeit konnte in beiden Fällen keine Hydrierungsreaktion festgestellt werden.

## Patentansprüche

1. Verfahren zur Herstellung von Alkylenglykoldialkylethern aus Alkylenglykolmonoalkylethern mit einer sekundären Hydroxylgruppe, dadurch gekennzeichnet, daß ein Alkylenglykolmonoalkylether der nachstehenden Formel I

$$R^1O(CH_2\underset{R^2}{\underset{|}{C}}HO)_n-CH_2\underset{R^3}{\underset{|}{C}}HOH \quad ,$$

worin $R^1$ ein Alkylrest mit 1 bis 25 C-Atomen ist, $R^2$ H, $CH_3$ oder $C_2H_5$ ist und innerhalb der Kette des Polyoxalkylenrestes, statistisch oder in Blöcken angeordnet, auch alle drei Bedeutungen annehmen kann, $R^3$ $CH_3$ oder $C_2H_5$ ist und n 1 bis 100 bedeutet,
in Gegenwart von Nickel auf einem Träger als Katalysator mit Wasserstoff bei Atmosphärendruck umgesetzt und aus dem Reaktionsprodukt der gebildete Alkylenglykoldialkylether gewonnen wird.

2. Verfahren nach Anspruch 1, dadurch gekennzeichnet, daß ein Alkylenglykolmonoalkylether der Formel I eingesetzt wird, worin $R^1$ ein Alkylrest mit 4 bis 18 C-Atomen ist, $R^2$ H oder $CH_3$ ist und innerhalb der Kette des Polyoxalkylenrestes, statistisch oder in Blöcken angeordnet, auch beide Bedeutungen annehmen kann, $R^3 = CH_3$ und n = 5 bis 50 ist.

3. Verfahren nach Anspruch 1 oder 2, dadurch gekennzeichnet, daß ein Nickel-Trägerkatalysator aus 5 bis 80 Gew.-% Nickel auf einem Trägermaterial verwendet wird, Gewichtsprozente bezogen auf den gesamten Katalysator, und soviel Nickel-Trägerkatalysator eingesetzt wird, daß 0,5 bis 15 Gew.-% Nickel, bezogen auf die umzusetzende Menge Alkylenglykolmonoalkylether, anwesend sind.

4. Verfahren nach einem oder mehreren der Ansprüche 1 bis 3, dadurch gekennzeichnet, daß Hydrierwasserstoff in einer Menge von 30 bis 500 Liter pro Kilogramm Alkylenglykolmonoalkylether pro Stunde eingesetzt wird und die Umsetzung bei einer Temperatur von 150 bis 300 °C durchgeführt wird.

5. Verfahren nach Anspruch 1, dadurch gekennzeichnet, daß der Alkylenglykolmonoalkylether und soviel von einem Nickel-Trägerkatalysator, bestehend aus 30 bis 65 Gew.-% Nickel auf einem Trägermaterial, Gewichtsprozente bezogen auf den gesamten Katalysator, in einem mit Rückflußkühler mit Wasserabscheider ausgestatteten Reaktionsgefäß vorgelegt werden, daß 1 bis 10 Gew.-% Nickel, bezogen auf Alkylenglykolmonoalkylether, anwesend sind, die vorgelegte Mischung auf eine Temperatur von 180 bis 250 °C erhitzt wird, durch die erhitzte Mischung 50 bis 250 Liter Wasserstoff pro Kilogramm Alkylenglykolmonoalkylether pro Stunde hindurchgeleitet werden bis das Reaktionsprodukt die angestrebte niedrige Hydroxylzahl aufweist, wobei der Wasserstoffüberschuß durch den Rückflußkühler abgezogen wird, und der gebildete Alkylenglykoldialkylether vom eingesetzten Katalysator abgetrennt wird.

## Claims

1. A process for the preparation of alkylene glycol dialkyl ethers from alkylene glycol monoalkyl ethers having a secondary hydroxyl group, which comprises reacting an alkylene glycol monoalkyl ether of the formula I

$$R^1O(CH_2\underset{R^2}{\underset{|}{C}}HO)_n-CH_2\underset{R^3}{\underset{|}{C}}HOH$$

in which $R^1$ is an alkyl radical having 1 to 25 carbon atoms, $R^2$ is H, $CH_3$ or $C_2H_5$ and; within the chain of the polyoxyalkylene radical, arranged randomly or in blocks, can also assume all three meanings, $R^3$ is $CH_3$ or $C_2H_5$ and n denotes 1 to 100,
with hydrogen at atmospheric pressure in the presence of nickel on a support as catalyst and recovering the alkylene glycol dialkyl ether formed from the reaction product.

2. The process as claimed in claim 1, wherein an alkylene glycol monoalkyl ether of the formula I is employed, in which $R^1$ is an alkyl radical having 4 to 18 carbon atoms, $R^2$ is H or $CH_3$ and, within the chain of the polyoxyalkylene radical, arranged randomly or in blocks, can also assume both meanings, $R^3$ is $CH_3$ and n is 5 to 50.

3. The process as claimed in claim 1 or 2, wherein a supported nickel catalyst containing 5 to 80 % by weight of nickel on a support material is used, per cent by weight being relative to the total catalyst, and sufficient supported nickel catalyst is employed that 0.5 to 15 % by weight of nickel, relative to the amount of alkylene glycol monoalkyl ether to be reacted, are present.

4. The process as claimed in one or more of claims 1 to 3, wherein the hydrogen for hydrogenation is employed in an amount from 30 to 500 liters per kilogram of alkylene glycol monoalkyl ether per hour and the reaction is carried out at a temperature of 150 to 300 °C.

5. The process as claimed in claim 1, wherein the alkylene glycol monoalkyl ether and such an amount of supported nickel catalyst, consisting of 30 to 65 % by weight of nickel on a support material, per cent by weight being relative to the total catalyst, are initially introduced into a reaction vessel equipped with a reflux condenser with water separator, that 1 to 10 % by weight of nickel, relative to alkylene glycol monoalkyl ether, are present, the initially introduced mixture is heated to a temperature of 180 to 250 °C, 50 to 250 liters of hydrogen per kilogram of alkylene glycol monoalkyl ether per hour are passed through the heated mixture until the reaction product has the desired low hydroxyl number, the excess of hydrogen being removed through the reflux condenser, and the alkylene glycol dialkyl ether formed is separated off from the catalyst employed.

## Revendications

1. Procédé pour préparer des éthers dialkyliques d'alkylène-glycols à partir d'éthers mono-alkyliques d'alkylène-glycols renfermant un radical hydroxy secondaire, procédé caractérisé en ce qu'on fait réagir avec de l'hydrogène sous la pression atmosphérique un monoétheralkylique d'alkylène-glycol répondant à la formule I suivante :

$$R^1O(CH_2\underset{R^2}{\underset{|}{CH}}O)_n-CH_2-\underset{R^3}{\underset{|}{CH}}OH$$

dans laquelle $R^1$ représente un radical alkyle contenant de 1 à 25 atomes de carbone, les $R^2$ représentent H, $CH_3$ ou $C_2H_5$ et peuvent également prendre les trois significations dans la chaîne du radical poly-(oxy-alkylène) avec une répartition statistique ou en séquences, $R^3$ représente $CH_3$ ou $C_2H_5$ et n désigne un nombre de 1 à 100,
en présence de nickel sur un support, comme catalyseur, et on isole du produit réactionnel l'éther dialkylique d'alkylène-glycol formé.

2. Procédé selon la revendication 1 caractérisé en ce qu'on utilise un monoéther alkylique d'alkylène-glycol de formule I dans lequel $R^1$ représente un alkyle contenant de 4 à 18 atomes de carbone, les $R^2$ représentent H ou $CH_3$ et peuvent également prendre les deux significations dans la chaîne du radical poly-(oxy-alkylène) avec une répartition statistique ou en séquences, $R^3$ représente $CH_3$ et n désigne un nombre de 5 à 50.

3. Procédé selon l'une des revendications 1 et 2, caractérisé en ce qu'on utilise un catalyseur au nickel sur support constitué de 5 à 80% en poids de nickel sur une matière support, par rapport au poids du catalyseur total, et en ce qu'on met en jeu une quantité du catalyseur constitué de nickel sur support suffisante pour qu'il y ait de 0,5 à 15% en poids de nickel par rapport à la quantité du mono-éther alkylique d'alkylène-glycol à faire réagir.

4. Procédé selon l'une quelconque des revendications 1 à 3, caractérisé en ce que l'hydrogène pour l'hydrogénation est mis en jeu en une quantité de 30 à 500 litres par kilogramme du mono-éther alkylique d'alkylèneglycol et par heure et en ce que la réaction est effectuée à une température de 150 à 300°C.

5. Procédé selon la revendication 1 caractérisé en ce qu'on place dans un récipient réactionnel équipé d'un réfrigérant à reflux avec séparateur d'eau, le mono-éther alkylique d'alkylène-glycol et une quantité d'un catalyseur au nickel sur support constitué de 30 à 65% en poids de nickel sur une matière support, par rapport au poids du catalyseur total, suffisante pour qu'il y ait de 1 à 10% en poids de nickel, par rapport au mono-éther alkylique d'alkylène-glycol, on chauffe le mélange préalablement mis en place à une température de 180 à 250°C, on fait passer à travers le mélange chauffé de 50 à 250 litres d'hydrogène par kilogramme du mono-éther alkylique d'alkylène-glycol et par heure jusqu'à ce que le produit réactionnel ait le faible indice d'hydroxyle souhaité, l'excès d'hydrogène étant retiré par le réfrigérant à reflux, et on sépare du catalyseur mis en jeu l'éther dialkylique d'alkylène-glycol formé.